# EUROPEAN PATENT SPECIFICATION

(11) **EP 4 284 275 B1**
(45) Date of publication and mention of the grant of the patent: **09.04.2025**
(21) Application number: 22705268.5
(22) Date of filing: 01.02.2022
(51) Int. Cl.: A61B 18/08, A61B 18/12

(54) **E-CUT SEALER-DIVIDER**
E-CUT-VERSIEGELER-TEILER
DISPOSITIF DE SCELLAGE-DIVISEUR À COUPE ÉLECTRONIQUE

(30) Priority: 01.02.2021 US 202163144055 P
(43) Date of publication of application: 06.12.2023
(60) Divisional application: 25158136.9
(73) Proprietor: Bolder Surgical, LLC, Marlborough, MA 01752 (US)
(72) Inventor: SCHMALTZ, Dale, Marlborough, MA 01752 (US); HART, Keir, Marlborough, MA 01752 (US); HUANG, Miao, Marlborough, MA 01752 (US); KENNEDY, Jenifer, Marlborough, MA 01752 (US); KRAMER, Dodge, Marlborough, MA 01752 (US); ROSS, David, Marlborough, MA 01752 (US); WARD, Arlen, Marlborough, MA 01752 (US)
(74) Representative: Gillespie, Richard
(86) International application number: PCT/US2022/014803
(87) International publication number: WO 2022/165444

(56) References cited:
- WO-A1-2019/006068
- US-A1- 2014 005 667
- US-A1- 2021 196 354
- US-B2- 9 566 108

## Description

### FIELD

This invention is related to surgical instruments. Specifically, but not intended to limit the invention, embodiments of the invention are related to surgical instruments for sealing and dividing tissue.

### BACKGROUND

In the last twenty years or more, the medical device industry has strived to provide reliable means to seal and divide tissue, including blood vessels. In some cases, vessel sealers have been offered that require the surgeon to repeatedly switch between a sealer and a cutting device. This method requires the surgeon first insert a sealer into the area, such as through a lumen, to seal tissue, then remove the device, and then re-enter with a cutting device. This method slows surgical procedures, so it is preferable to provide the sealing and dividing capabilities in a single device. Others require multiple activation steps, which increase the risk of error.

Some instruments provide a vessel sealer in combination with a mechanical (knife) cutting mechanism. Other devices (such as those disclosed in WO 2018/006068 and US 2021/196354) provide harmonic seal and cut operations, which also employ mechanical energy. For years, the industry has attempted without significant success to provide a vessel sealer with electronic (e.g., radiofrequency energy) cutting capability.

### SUMMARY

The present disclosure is directed towards a surgical system for sealing and severing tissue, the features of which are set out in the appended claims. In one embodiment of the inventions, a surgical system for sealing and severing tissue is disclosed. The system comprises a generator configured to output radiofrequency (RF) energy, including delivering a first RF power signal during a seal cycle, and delivering a second RF power signal during a cut cycle, and a surgical device electrically coupled to the generator output. The device comprises an elongate shaft; an end effector coupled to a distal end portion of the elongate shaft and defining a longitudinal axis, the end effector comprising first and second jaws configured to approximate each other in a closed position for compressing tissue extending therebetween and disposed transverse to the longitudinal axis, the first and second jaws having respective opposing conductive seal surface electrodes configured for sealing spaced apart first and second portions of the compressed tissue when the first RF power signal is conducted through a circuit including the respective seal surface electrodes during a seal cycle; and a cut electrode disposed within an interior region of the first jaw, wherein the cut electrode is aligned with the longitudinal axis and has an elongate conductive edge surface, the cut electrode having a height profile relative to the first jaw such that, when the first and second jaws are in the closed position compressing the tissue, the elongate conductive edge surface of the cut electrode presses against a third portion of the tissue located between the spaced apart first and second portions. The cut electrode is configured to sever the third portion of the tissue when the second RF power signal is conducted through a circuit including the cut electrode and a respective one of the seal surface electrodes during a cut cycle performed after the seal cycle is completed.

Optionally, the first RF power signal of the surgical system imparts a sealing power of no more than 55 Watts, with a current of no more than 2.5 Amperes rms, and a maximum voltage of 110 Volts rms. Optionally, the second RF power signal of the surgical system imparts a cutting power of no more than 80 Watts, with a current of no more than 2.5 Amperes rms, and a maximum voltage of 380 Volts rms.

In various embodiments of the surgical system, the generator maintains substantially constant output power by varying current or voltage in response to changes in impedance of the tissue during respective seal and cut cycles.

In some embodiments of the surgical system, wherein, when the first and second jaws are in the closed position compressing the tissue, the elongate conductive edge surface of the cut electrode stretches the third portion of the tissue.

In some embodiments, the surgical system further comprises a resilient member disposed in an interior region of the second jaw opposing the elongate conductive edge surface of the cut electrode, wherein when the first and second jaws are in the closed position compressing the tissue, the elongate conductive edge surface of the cut electrode and the resilient member are configured to compress the third portion therebetween. Optionally, the resilient member has a bias against the elongate conductive edge surface of the cut electrode so as to impose a restoring force that presses the third portion of the tissue against the elongate conductive edge surface of the cut electrode so as to maintain contact of the third portion of the tissue against the elongate conductive edge surface of the cut electrode during a cut cycle. Optionally, the resilient member comprises an elastomer. In some embodiments, when the first and second jaws are in the closed position compressing the tissue, a portion of the elastomer is deformed into an elastomer reservoir defined by the second jaw. Optionally, the resilient member comprises a non-conductive support surface that contacts the third tissue portion.

In some embodiments, the cut electrode is shaped and configured to uniformly distribute a current concentration along the elongate conductive edge surface during a cut cycle. Optionally, the elongate conductive edge surface of the cut electrode has a convex rounded cross-sectional profile.

In some embodiments, the respective seal surface electrode that forms the cut cycle circuit with the cut electrode comprises a seal surface profile, the seal surface profile having a tissue compression portion and a first rounded edge portion, the first rounded edge portion configured to minimize current concentrations during a cut cycle. 14. Optionally, the respective conductive seal surface electrodes of the first and second jaws impart a bi-polar effect on the respective first and second portions of the tissue during a seal cycle. Optionally, the respective seal surface electrode that forms the cut cycle circuit with the cut electrode acts as a dispersive electrode such that the cut electrode imparts a monopolar effect on the third portion of the tissue during a cut cycle.

In some embodiments, the interior region of the first jaw defines at least one tissue reservoir disposed adjacent the cut electrode, wherein the tissue reservoir is configured to receive prolapsed tissue when the first and second jaws are in the closed position compressing the tissue. Optionally, the at least one tissue reservoir comprises a first tissue reservoir disposed adjacent a first side of the cut electrode, and a second tissue reservoir disposed adjacent a second side of the cut electrode opposite the first side.

In some embodiments, the end-effector has an envelope diameter of no more than eight millimeters when the first and second jaws are in the closed position.

In some embodiments, the cut cycle is no more than 1.5 seconds in duration.

In various embodiments, the generator is configured to vary one or both of a current and voltage of the first RF signal up to a predetermined maximum seal current in response to changes in impedance of the first and second tissue portions during a seal cycle, and the generator is configured to vary one or both of a current and voltage of the second RF signal up to a predetermined maximum cut current in response to changes in impedance of the third portion of the tissue during a cut cycle.

In some embodiments, the first RF power signal is maintained at a substantially constant power during the seal cycle, and the second RF power signal is maintained at a substantially constant power during the cut cycle. The substantially constant power level of the second RF power signal is greater than the substantially constant power level of the first RF power signal.

In some embodiments, the tissue comprises a blood vessel.

In some embodiments, the respective conductive seal surface electrodes of the first and second jaws impart a bi-polar effect on the respective first and second portions of the tissue during delivery of the first RF power signal.

### BRIEF DESCRIPTION ON THE DRAWINGS

FIG. 1 is a perspective view of a medical system according to embodiments of the disclosed inventions;
FIGS. 2A-2B are side views of the end portion of the system of FIG. 1;
**FIG.** 3 is a perspective view of the end portion of the system of FIG. 1;
FIG. 4 is a cross-sectional view of the end portion components of the system of FIG. 1;
FIGS. 5A-5B are cross-sectional views of components of a first jaw in the device illustrated in FIG. 4;
FIGS. 5C-5D are cross-sectional views of components of alternative embodiments of the first jaw of the medical device;
FIGS. 6A-6B are cross-sectional views of components of a second jaw in the device illustrated in FIG. 4;
FIG. 6C is cross-sectional view of components of an alternative embodiment of the second jaw of the medical device
**FIG.** 7 is a cross sectional view illustrating tissue effects of the device of FIGS. 1-6C;
FIGS. 8A-8B illustrate cross-sectional views of cut electrodes and current concentration for comparison;
FIG. 9 is a flow chart of a method of use of the system of FIGS. 1-6C;
**FIG.** 10 is a flow chart of an alternative method of use of the system of FIGS. 1-6C;
**FIGS.** 11A-E are cross-sectional views of alternative embodiments of the system of FIGS. 1-8A;
FIG. 12 is a graph of an exemplary seal cycle, according to embodiments of the disclosed inventions;
FIG. 13 is a graph of two exemplary cut cycles, according to embodiments of the disclosed inventions; and
FIG. 14 is a graph of a detailed rescaled view of the cut cycles of FIG. 13.

### DETAILED DESCRIPTION

For the following defined terms, these definitions shall be applied, unless a different definition is given in the claims or elsewhere in this specification.

**All** numeric values are herein assumed to be modified by the term "about," whether or not explicitly indicated. The term "about" generally refers to a range of numbers that one of skilled in the art would consider equivalent to the recited value (i.e., having the same function or result). In many instances, the terms "about" may include numbers that are rounded to the nearest significant figure.

**The** recitation of numerical ranges by endpoints includes all numbers within that range (e.g., 1 to 5 includes 1, 1.5, 2, 2.75, 3, 3.80, 4, and 5).

**As** used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

Various embodiments are described hereinafter with reference to the figures. The figures are not necessarily drawn to scale, the relative scale of select elements may have been exaggerated for clarity, and elements of similar structures or functions are represented by like reference numerals throughout the figures. It should also be understood that the figures are only intended to facilitate the description of the embodiments, and are not intended as an exhaustive description of the invention or as a limitation on the scope of the invention, which is defined only by the appended claims and their equivalents. In addition, an illustrated embodiment needs not have all the aspects or advantages shown. An aspect or an advantage described in conjunction with a particular embodiment is not necessarily limited to that embodiment and can be practiced in any other embodiments even if not so illustrated.

Before providing a more detailed description of the inventions and embodiments disclosed herein, it is expedient to describe some of the challenges faced when attempting to provide a reliable tissue sealer-divider.

**The** first problem is providing a reliable, safe tissue seal and addressing some biases in the medical device industry. For many years, it was believed that a very high power, often 100 Watts or more, was required to achieve tissue sealing. At such high powers, traditional devices were/are required to include a myriad of safety features to prevent tissue from being charred and/or preventing thermal spread - or damage to adjacent tissue. Some devices do so by rapidly turning the devices on and off. See, for example, US Pat. No. 6,228,080, issued on May 8, 2001 to Gines, which discloses a method of cycling a power application to tissue to prevent tissue necrosis, which is caused by the build-up of heat near tissue being treated. Power cycling, however, has its own side-effects, with one being the demand it places on programing and the power supply and hardware involved. The Applicant has overcome these and other problems in vessel sealing by providing a low power tissue sealing device, such as the one disclosed in commonly-owned US Pat. No. 10,166,064, issued on January 1, 2019, and related applications, and commonly owned US Pat. No. 10,342,599, issued on July 9, 2019.

The second problem is providing a safe, reliable cut after tissue is sealed, to separate the tissue. Historically, surgeons would remove the tissue sealing device from the patient after sealing, then insert a cutting device. This was cumbersome and extended the duration of surgeries. Vessel sealers with mechanical dividers have been developed to reduce the duration of surgeries. These devices typically function by moving a knife through sealed tissue to divide it. One exemplary device is shown and described in commonly-owned US Pat. No. 10,765,471, issued on September 8, 2020, and related applications.

Such devices, however, limit flexibility in seal surface geometry and jaw manipulation because a knife must pass through the jaws. For example, these devices prevent or limit use with an articulating device and/or in a catheter setting.

Generally, it is desirable to limit the number of times a surgeon must manipulate the device, such as by providing a single-action device that performs two functions in response to a single action by a surgeon. In knife-based cutting systems, however, single-action operation may introduce the risk of a surgeon moving his or her hand between the seal and cut actions; on the other hand, automatic knife-actuation has the inherent risk of actuating the knife when the jaws are not closed, and the moving knife may also become lodged in the tissue or jaws, which requires an involved process to remove. It is therefore desirable to provide a device that is both "single action" in nature and overcomes the problems described above.

In an attempt to overcome these problems, numerous entities have attempted to develop what is known in the industry as e-cutting devices, such as the PKS^{™} OMNI^{™} device sold by Olympus (see e.g. www.olympusamerica.com). The PKS OMNI device, along with the related G400^{™} generator, applies high power in a cycling manner to seal tissue between the jaws.

However, none of the known devices currently available on the market can claim a 100% cut performance. Instead, the devices typically only cut "most" of the tissue that is intended to be cut, but leave what is known in the industry as "tags" which must be cut separately by the surgeon, thus defeating the purpose of a "single action" device.

The Applicant has tested embodiments described herein and confirmed that embodiments described herein achieve a 100% cut performance, which is the highest benchmark possible. This 100% cut performance is in combination with low thermal spread, which the Applicant has proven to provide both a stronger seal and improved patient outcomes.

Embodiments of the present invention overcome the above-stated problems while providing other new and useful features.

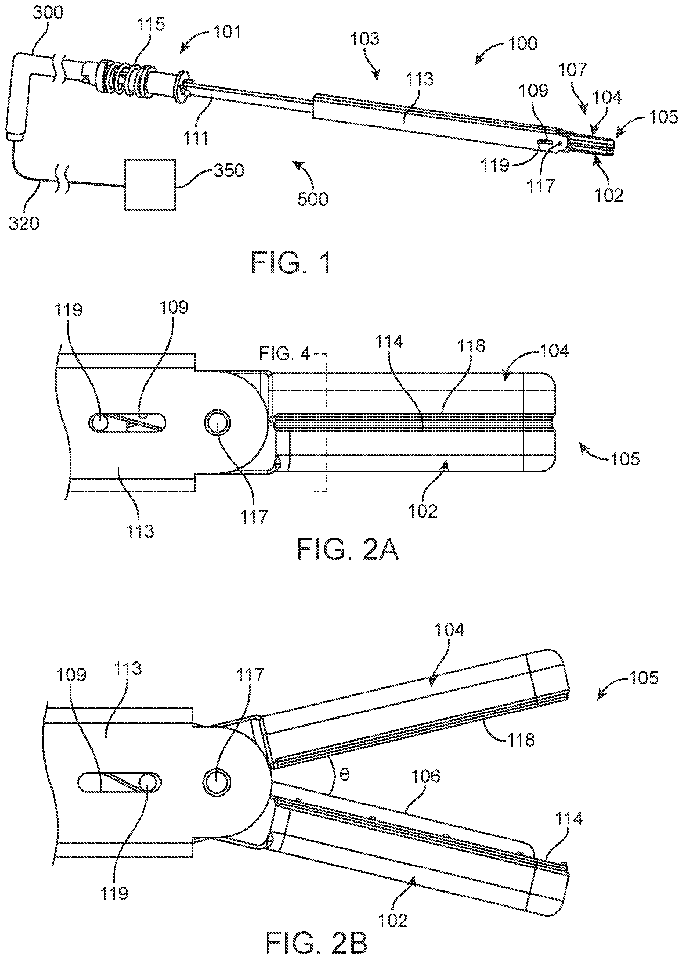

**FIG. 1** is a perspective view of a medical system 500, in accordance with embodiments of the disclosed inventions. The system 500 includes a device 100 having a proximal portion 101, a body portion 103 and a distal portion 107. The proximal portion 101 of the device is configured to be coupled to an actuator 300, such as a handle, a laparoscopic system, a surgical robotic system or the like. The body portion 103 of the medical device 100 comprises an inner tubular member 111 and an outer tubular member 113, where the inner tubular member 111 is configured to translate relative to the outer tubular member 113. The distal portion 107 of the medical device 100 comprises an end effector 105 having a first jaw 102 and a second jaw 104. The actuator 300 is configured to actuate the medical device 100, control physical movements (e.g., open and close jaws 102, 104), engage the end effector 105 with tissue of a patient, and electrically engage the end effector 105 to seal and divide tissue. An electrical wire or wires 320 extend from a power source 350, such as a radio frequency generator, through the actuator 300 to the end effector 105; the electrical wires 320 are configured to deliver energy to the end effector 105. In an alternative embodiment, the electrical wires extend from the actuator 300 to the end effector 105, and the connection of the medical device 100 to the power source 350 is wireless. The energy delivered may be radio-frequency (RF) energy or any other suitable energy for sealing and cutting tissue.

Referring back to the proximal portion 101 of the medical device 100, the proximal portion 101 includes a spring cartridge 115 (or other suitable sealing mechanism) configured to exert a spring-limited sealing force when actuated by the actuator 300, thereby compressing the spring and proximately translating (e.g., pulling) the inner tubular member 111. The inner tubular member 111 of the medical device 100 comprises a bolt or cam pin 119 slidably disposed within a slot 109 of the outer tubular member 113 **(****FIGS. 1-2B****).** The cam pin 119 is fixedly attached to the inner tubular member 111 and is configured to transfer forces to the end effector 105, as the inner tubular member 111 longitudinally translates relative to outer tubular member 113, allowing closing **(****FIG. 2A****)** or opening **(****FIG. 2B****)** of the jaws 102 and 104. The slot 109 of the outer tubular member 113 is configured to allow movement of the cam pin 119 of the inner tubular member 111 without transferring forces to the outer tubular member 113. The slot 109 of the outer tubular member 113 of the medical device 100 minimizes or avoids undesirable motion of the jaws 102 and 104 (e.g., rotation). The distal portion 107 of the medical device 100 further comprises a pivot pin 117 disposed between the jaws 102 and 104. The pivot pin 117 is configured to serve as a bearing surface for the jaws 102 and 104 and as alignment for the components of the end effector 105.

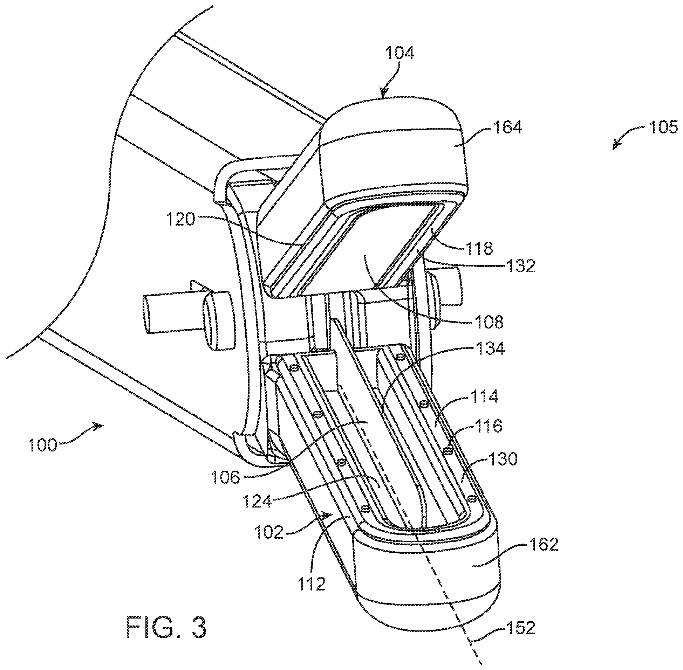

By way of further illustration, **FIGS. 2A-B** depict the end effector 105 of the medical device 100 of the system 500, having jaws 102 and 104 in a closed and opened configuration, respectively. As shown in **FIG. 2A****,** the cam pin 119 slidably is disposed within the slot 109 of the outer tubular member 113 and proximately located when the jaws 102 and 104 are in a closed configuration. **FIG. 2B** shows the cam pin 119 is distally located within the slot 109 of the outer tubular member 113 when the jaws 102 and 104 are in an opened configuration. Both jaws 102 and 104 move relative to each other to form the opened configuration of the end effector 105 of the medical device 100. In the opened configuration, the jaws 102 and 104 are configured to separate from each other in a range of 20 to 100 degrees. **FIG. 2B** illustrates an exemplary opened configuration having an angle θ of approximately 25° between jaws 102 and 104. In some embodiments, the angle θ of approximately 60° between jaws 102 and 104 (not shown). In some embodiments, the length of the respective jaws 102 and 104 are in a range of 15 to 23 millimeters long, where the length is taken from the pivot pin 117 to the distal ends of each jaw (e.g., non-traumatic distal end portions 162 and 164 better shown in **FIG. 3****).** In some embodiments, the opened configuration of the jaws 102 and 104 have a working range of 14 to 23 millimeters long; where the working range is the length between the distal ends of each jaw in an opened configuration (e.g., distance between the distal end portions 162 and 164 of **FIG. 3****).** In an alternative embodiment, only one of the jaw move relative to the other jaw (not shown). Additionally, **FIGS. 2A-B** depicts conductive core members 114, 118, and **FIG. 2B** depicts a cut electrode 106, which will be described in further detail below.

**FIG. 3** illustrates a perspective view of the end effector 105 of the medical device 100 of the system 500 in an opened configuration. The jaws 102 and 104 comprise a substantially elongated straight configuration, whereby the first jaw 102 extends along a longitudinal axis 152. The jaws 102 and 104 further comprise respective curved, rounded, and/or non-traumatic distal end portions 162 and 164. In alternative embodiments, the jaws 102 and 104 may comprise an elongated curved configuration (not shown), such as with a Maryland-style curve. In some embodiments, the jaws 102 and 104 are coupled to a flexible shaft (not shown) in the medical device 100.

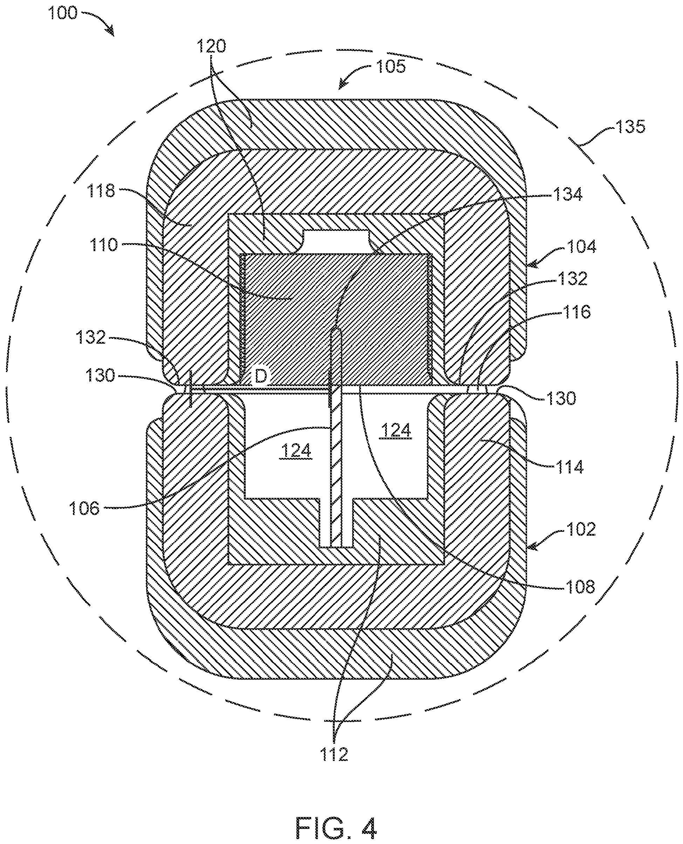

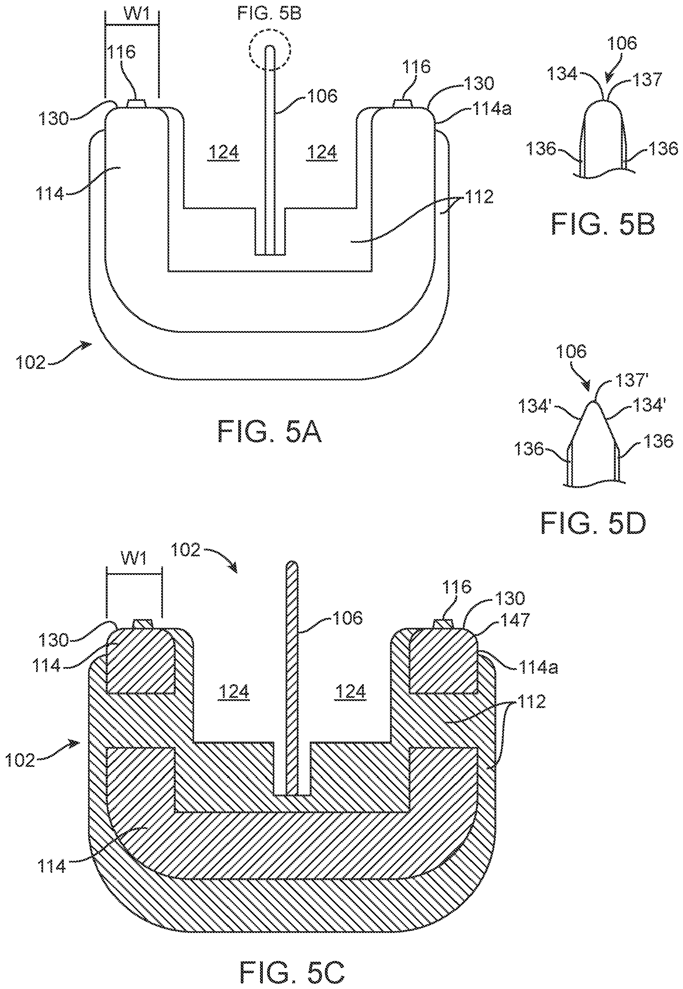

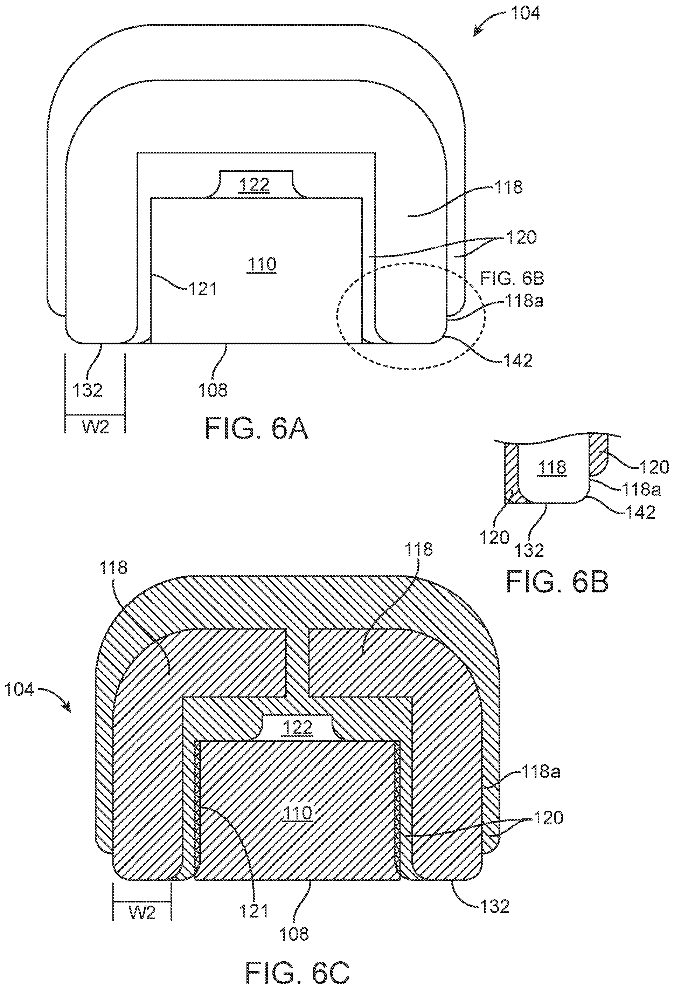

**The** jaws 102 and 104 comprise respective conductive core members 114 and 118, which are configured to provide electrically conductive paths to the end effector 105. The conductive core members 114 and 118 are composed of stainless steel or any other suitable conductive material. The conductive core members 114 comprises a seal surface 130 of the jaw 102, and the conductive core members 118 comprises a seal surface 132 of the jaw 104. The seal surfaces 130 and 132 are disposed along the perimeter of their respective jaw 102 and 104, including along the curved distal end portions 162 and 164 of the jaws, as shown in **FIG. 3****.** The seal surfaces 130 and 132 are configured to contact and seal tissue, such as when the end effector 105 of the device 100 is in a closed configuration. The first jaw 102 further comprises an insulation element 112 disposed around and covering the conductive core member 114, except for the seal surface 130, as better appreciated in **FIGS. 4****,** **5A** and **5C****.** The second jaw 104 further comprises an insulation element 120 disposed around and covering the conductive core member 118, except for the seal surface 132, as better appreciated in **FIGS. 4****,** **6A** and **6C****.** The seal surfaces 130 and 132 are exposed (e.g., without insulation) to provide electrically conductive paths (e.g., bipolar) and seal tissue disposed between the seal surfaces 130 and 132. The seal surface 130 includes one or more non-conductive stop members 116 composed of ceramic or any other suitable non-conductive material, to prevent direct contact between the seal surfaces 130 and 132 avoiding short outs, in a manner known to those skilled in the art. In some embodiments, the seal surface 132 may also include one or more non-conductive stop members 116.

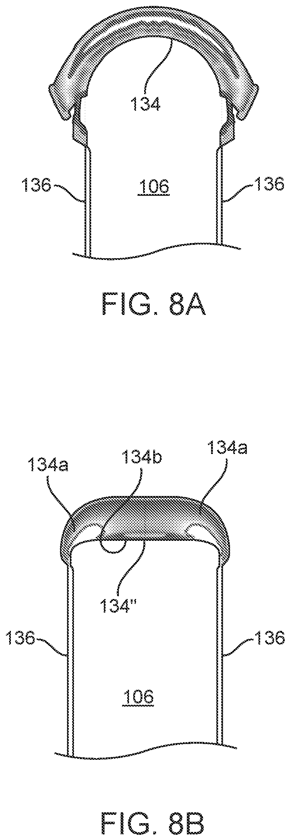

**The** first jaw 102 further comprises a cut electrode 106 and at least one tissue reservoir 124. As shown in **FIG. 3****,** the cut electrode 106 is disposed within a tissue reservoir 124 and comprises an elongated blade-like configuration extending along the longitudinal axis 152 of the first jaw 102. The cut electrode 106 includes an elongated profile or edge 134, where the edge 134 is substantially curved, rounded or any other suitable configuration. The curved edge 134 of the cut electrode 106 is desirable to provide a relatively large cutting surface area and more uniform current density for tissue cutting **(****FIGS. 5B** and **8A****).** As better appreciated in **FIG. 5B****,** the cut electrode 106 includes a non-conductive material 136, such as a coating or insulation, except for the edge 134. The conductive edge 134 of the cut electrode 106 is configured to provide uniform current density for tissue cutting, as it will be described in further detail below.

**The** elongated cut electrode 106 comprises a width in the range of 0.25 to 1.50 millimeters, sufficiently narrow to concentrate current to cut tissue near, adjacent or in contact with the edge 134, yet wide enough to prevent an inadvertent mechanical cut of the tissue when compressed between the jaws 102 and 104 prior to the cut cycle. The cut electrode 106 is further configured to avoid or minimize deformation of the cut electrode 106 and/or edge 134 during use of the end effector 105 (e.g., seal and cut cycles). The elongated cut electrode 106 is fixedly disposed in the first jaw 102. The elongated cut electrode 106 comprises a height profile wherein the edge 134 is disposed above or extending beyond the profiles of the seal surface 130 and stop members 116 **(****FIGS. 3-5A**, **5C**, **7** and **11A-E**). Optionally, those skilled in the art will also recognize that a moving cut electrode (e.g., slidably disposed) may be suitable for some applications.

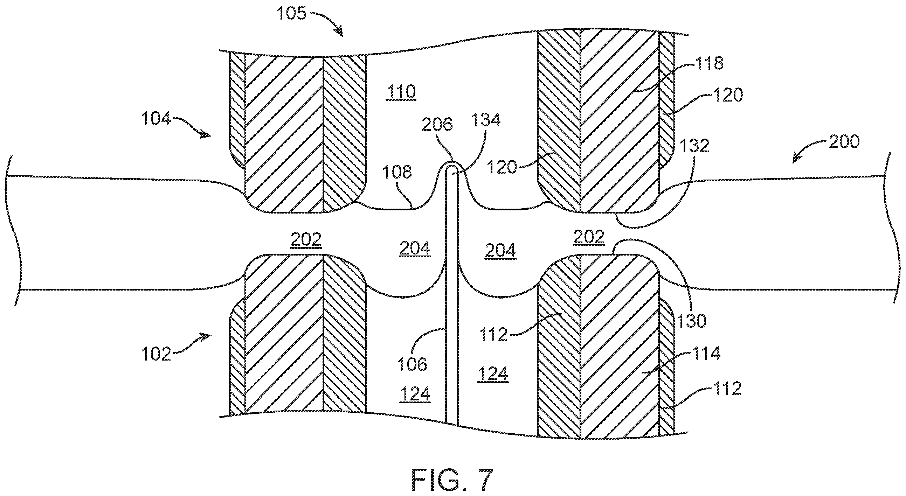

In some embodiments, the tissue reservoir 124 is disposed on both sides along the length of the cut electrode 106 **(****FIGS. 3-5A**, **5C**, **7** and **11A-E**). The tissue reservoir 124 is configured to receive tissue that is displaced or prolapsed during the closed configuration of the end effector 105 and/or during cutting (e.g., cut cycle). The tissue reservoir 124 is further configured to allow the end effector 105 to obtain and/or maintain its closed configuration when tissue is grasped, held and/or retained between the jaws 102 and 104, such that tissue is displaced to the tissue reservoir 124, as it will be further described in **FIG. 7****.** The tissue reservoir 124 also is further configured to enhance compression on tissue between the seal surfaces 130 and 132.

The second jaw 104 of the end effector 105 comprises an elongated cavity (element 121 in **FIGS. 6A** and **6C****)** having a resilient member 110 therebetween. The resilient member 110 comprises an elastomer or any other suitable polymeric material having rubber-like elastic properties (e.g., silicone, fluoroelastomers, polyurethane, foams or the like). The resilient member 110 forms a non-conductive support surface 108, shown in **FIG. 3****,** where the support surface 108 is configured to contact the cut electrode 106 in the first jaw 102 and/or contact tissue disposed therebetween. In some embodiments, the non-conductive support surface 108 may be formed as a coating on the resilient member 110, or a different material from the resilient member 110. The non-conductive support surface 108 and/or resilient member 110 have elastic properties, deforming and restoring forces, such as, deforming when the cut electrode 106 and/or tissue are pushed against them (e.g., end effector 105 in a closed configuration). Additionally, the non-conductive support surface 108 and/or resilient member 110 exert restoring force (e.g., push, spring-like) on the tissue against the conductive edge 134 of the cut electrode 106 configured to maintain uniform contact of the tissue against the cut electrode 106 for e-cutting (e.g., cut cycle), when the end effector 105 is in the closed configuration.

In some embodiments, the non-conductive support surface 108 and/or resilient member 110 may be configured to extend, occupy or move (partially or substantially) within the tissue reservoir 124 of the jaw 102, such as, prior to application of the cut cycle (not shown). In some embodiments, the non-conductive support surface 108 and/or resilient member 110 is configured to give way during tissue sealing and to push back during tissue cutting to maintain an effective or sufficient pressure on tissue to be cut. In some embodiments, the non-conductive support surface 108 and/or resilient member 110 is provided in a pre-loaded formation (that is, the elastomer may be flush with the top of seal surface 132 to provide an immediate response to tissue during grasping of the tissue, and drive tissue into the reservoir 124). In alternative embodiments, the resilient member 110 may include a relatively hard plate supported by a spring (not shown).

In some embodiments, a durometer of the non-conductive support surface 108 and/or resilient member 110 may be selected to maintain tissue contact with the cut electrode 106 as the tissue is cut and/or exhibits varying thicknesses. In some embodiments, the durometer may be selected to not inhibit closure of the jaws 102, 104. In some embodiments, the non-conductive support surface 108 and/or resilient member 110 may have a Shore A durometer up to 40. In some embodiments, the Shore A durometer may be greater than 20. In some embodiments, the durometer may be selected to maintain tissue contact with the cut electrode 106 during the cut cycle without biasing the jaws apart.

**FIG. 4** illustrates a cross-sectional view of the end effector 105 of the medical device 100 of the system 500 in the closed configuration of **FIG. 2A****.** The end effector 105 comprises a pair of jaws, including the first jaw 102 and the second jaw 104. The jaws 102 and 104 are approximate one another, such as to grasp, hold and/or retain tissue therebetween, and move away from each other, such as to release tissue (not shown). The seal surfaces 130 and 132 of the respective first jaw 102 and second jaw 104 are configured for bipolar sealing of tissue disposed between the pair of jaws. The seal surfaces 130 and 132 seal tissue positioned therebetween by conducting bipolar energy through tissue grasped, held and/or retained between the jaws 102 and 104 during a seal cycle. In the bipolar sealing cycle, the current density is approximately the same between the seal surfaces 130 and 132, since the exposed/conductive area of the seal surfaces 130 and 132 are approximately the same. In some embodiments, the seal cycle of the medical device 100 comprises applying a sealing power of 50 Watts nominal or less. In some embodiments, the sealing power has a current of 2.5 Amperes or less.

In some embodiments, the first seal surface 130 is the only exposed portion of the conductive core member 114, and the second seal surface 132 is the only exposed portion of the conductive core member 118.

In some embodiments, the jaws 102 and 104 are composed, formed and/or made by their respective conductive core members 114 and 118. As shown in **FIG. 4****,** the conductive core members 114 and 118 are covered, coated, sandwiched and/or insulated with respective insulations 112 and 120, except for their respective seal surfaces 130 and 132, which are exposed and conductive. The seal surface 130 includes the one or more non-conductive stop members 116 composed of ceramic or any other suitable non-conductive material. The seal surfaces 130 and 132 are disposed along the perimeter of their respective jaw 102 and 104, as better appreciated in **FIG. 3****.**

In some embodiments, the end effector 105 is configured to seal tissue grasped, held or restrained between the jaws 102 and 104, then cut tissue. The seal and cut cycles are sequential, such that the tissue is first sealed, then cut. The end effector 105 comprises a distance between the cut electrode 106 and respective seal surfaces 130 and 132 **(****FIGS.** 3, **4**, **5A, 5C**, and **11A-E**). The distance, shown as distance "D" in **FIG. 4**, allows for a section of the tissue to be disposed between respective seal surfaces 130 and 132 and the cut electrode 106, such that after the tissue is sealed by the seal cycle of the device 100, the section of tissue would maintain some moisture to allow for current flow from the cut electrode 106 during the cut cycle of the device 100 to perform cutting of the tissue. Further, the distance "D", along with the force exerted by the non-conductive support surface 108 and/or resilient member 110 on the tissue when the end effector 105 is in a closed configuration, allows tissue movement into the tissue reservoir 124. Distance "D" combined with the dimensions of the tissue reservoir 124 allow for an adequate amount of tissue overlaying and adjacent to the cut electrode 106 to avoid being desiccated during the seal cycle. In some embodiments, the distant "D" ranges between 0.5 to 2 millimeters. If the distance "D" is less than 0.5 millimeters, the sealed portion of the tissue would be too close to the cut electrode 106 during the seal cycle, then the impedance of tissue between the cut electrode 106 and the seal surface 130 or 132 will be too high, which will adversely affect the cut cycle. Additionally, if the distance "D" is less than 0.5 millimeters, for example, the cut electrode 106 may not cut tissue but rather simply char tissue, because there is little or no low-impedance path. In other words, the device 100 may be configured to provide a low-impedance path from the cut electrode edge 134 through a relatively large area of low-impedance tissue 204 **(****FIG. 7****)** after tissue sealing; which may be referenced herein as a pillow. The resulting current concentration along the edge 134 of the cut electrode 106 during the cut cycle creates a "monopolar effect" as the current propagates through the relatively low-impedance tissue 204 to the respective one of the seal surfaces 130, 132 that is functioning as the dispersive electrode.

**As** further shown in **FIG. 4****,** the pair of jaws 102 and 104 of the closed configuration of the end effector 105 comprises an envelope diameter 135 of 8 millimeters or less. Those skilled in the art will recognize that the term "envelope diameter" references to a substantially circular space within which the entirety of an object (e.g., jaws 102 and 104) can fit, even if the object (e.g., end effector 105) is not round. In some embodiments, the envelope diameter 135 is 6 millimeters or less. In some embodiments, the envelope diameter 135 is 10 millimeters or less. In some embodiments, the envelope diameter 135 is 12 millimeters or less.

**FIGS. 5A-5D** illustrate cross-sectional views of the first jaw 102 and components, in accordance with embodiments of the disclosed inventions. As disclosed above, the conductive core member 114 is covered, coated, sandwiched and/or insulated with insulation 112, except for the seal surface 130, which is exposed/conductive. **FIG. 5A** depicts the first jaw 102 of **FIG. 4** where the conductive core member 114 comprises a continuous configuration (e.g., "U" shape cross-section). Alternatively, the conductive core member 114 may include sectional portions, as shown in **FIG. 5C****,** as long as the conductive core member 114 is electrically engaged by the actuator 300 coupled to the power source 350 and is configured to deliver energy via the seal surface 130.

The seal surface 130 of the conductive core member 114 of the first jaw 102 comprises a width "W1", as shown in **FIGS. 5A** and **5C****.** In some embodiments, the width "W1" ranges between 5 and 1 millimeter. If the width "W1" of the seal surface 130 is less than 2 millimeters is considered a narrow width.

**As** shown in **FIGS. 5A** and **5C****,** the cut electrode 106 is disposed within the tissue reservoir 124 and comprises the cut electrode edge 134; the edge 134 having a substantially curved, rounded, convex configuration, as better appreciated in **FIG. 5B****.** The cut electrode 106 includes a non-conductive material, coating or insulation 136, except for the edge 134 **(****FIG. 5B****)** and except for a portion coupling the cut electrode 106 with the power source 350 **(****FIG. 1****).** In some embodiments, the cut electrode 106 is insulated on all surfaces by insulation 136, leaving the cut electrode edge 134 exposed and the coupling with the power source 350 without insulation. The edge 134 further comprises a conductive engagement surface 137 **(****FIG. 5B****).** The conductive engagement surface 137 comprises a convex and smooth configuration to prevent current concentrations on the edge 134 of the cut electrode 106. The edge 134 of the cut electrode 106 may be curved laterally throughout the entire length of the cut electrode 106, as better appreciated in **FIG. 3****.** Alternatively, the edge 134 of the cut electrode 106 may be curved throughout longitudinal portions or sections of the cut electrode 106 (not shown) and/or comprises other suitable configurations **(****FIG. 5D). FIG. 5D** illustrates an alternative edge 134' of the cut electrode 106, where the edge 134' comprises a beveled configuration having a rounded conductive engagement surface 137' coupled to the beveled edges 134'. The cut electrode 106 includes a non-conductive material, coating or insulation 136, except for the beveled edges 134' and the rounded conductive engagement surface 137' **(****FIG. 5D****).**

Although, having a cut electrode 106 with rounded edge 134 and/or convex conductive engagement surface 137, as shown in **FIGS. 3-5D** may be considered counterintuitive (industry believes sharp edges on a cut blade would concentrate current and improve cutting ability), the curved edge 134 and/or convex conductive engagement surface 137 of the cut electrode 106 comprises a large radius, which creates a higher current concentration spread over surface area, thus providing improved cutting performance compared to a cut electrode with a sharp edge.

**FIGS. 6A-6C** illustrate cross-sectional views of the second jaw 104 and components, in accordance with embodiments of the disclosed inventions. As previously disclosed, the conductive core member 118 is covered, coated, sandwiched and/or insulated with insulation 120, except for the seal surface 132, which is exposed/conductive. **FIG. 6A** depicts the second jaw 104 of **FIG. 4** where the conductive core member 118 comprises a continuous configuration (e.g., "∩" shape cross-section). Alternatively, the conductive core member 118 may include sectional portions, as shown in **FIG. 6C****,** as long as the conductive core member 118 is electrically engaged by the actuator 300 coupled to the power source 350 and is configured to deliver energy via the seal surface 132.

As shown in **FIGS. 4**, **6A-6C** the insulation 120 disposed on an outer surface 118a of the conductive core member 118 substantially, but not fully, covers the outer surface 118a, as better appreciated in **FIG. 6B****,** exposing a conductive rounded edge portion 142 of the conductive core member 118. Thus, the insulation 120 disposed on an outer surface 118a of the conductive core member 118 is set back slightly, as depicted in **FIGS. 4**, **6A-6C**, from the rounded edge portion 142 to expose conductive core member 118. The rounded edge portion 142 extends longitudinally along the second jaw 104 and is configured to minimize any current concentration during the bipolar sealing cycle.

Referring back to first jaw 102, **FIGS. 4****,** **5A** and **5C** show the insulation 112 disposed in an outer surface 114a of the conductive core member 114 substantially, but not fully, covers the outer surface 114a, exposing a conductive rounded edge portion 147 of the conductive core member 114. The rounded edge portion 147 extends longitudinally along the first jaw 102 and is configured to minimize current concentration and subsequent tissue effect.

In alternative embodiments, the insulations 112 and 120 fully cover their respective outer surface 114a and 118a of the conductive core members 114 and 118, thus, the rounded edge portions 142 and 147 are not exposed (not shown).

The seal surface 132 of the conductive core member 118 of the second jaw 104 comprises a width "W2", as shown in **FIGS. 6A** and **6C****.** In some embodiments, the width "W2" ranges between 5 and 1 millimeter. If the width "W2" of the seal surface 130 is less than 2 millimeters then W2 is considered a narrow width. In some embodiments, the respective seal surfaces 130 and 132 have widths W1 and W2 of 3 millimeters or less each. In some embodiments, the respective seal surfaces 130 and 132 have widths W1 and W2 of two millimeters or less each. In some embodiments, at least a portion of at least one seal surface 130, 132 has width W1, W2 of 3 millimeters or less. In some embodiments, at least a portion of at least one seal surface 130, 132 has width W1, W2 of 2 millimeters or less. In some embodiments, at least a portion of at least one seal surface 130, 132 is a narrow seal surface.

**The** second jaw 104 comprises a cavity 121 having the resilient member 110 disposed therebetween **(****FIGS. 6A** and **6C****).** The cavity 121 comprises a relief space 122 for the resilient member 110 to occupy when the resilient member 110 is deformed by the cut electrode 106 and/or tissue such as when the end effector 105 in a closed configuration. In some embodiments, the resilient member 110 may deform and at least partially occupy the relief space 122 prior to the application of the cut cycle, such as during a seal cycle, or such as prior to a seal cycle as the end effector 105 is in a closed configuration **(****FIGS. 2A** and **4****).** The relief space 122, shown in **FIGS. 6A** and **6C****,** is configured to prevent the resilient member 110 from cupping, whereby a gap forms between the cut electrode 106 and tissue disposed between the cut electrode 106 and the resilient member 110. Therefore, the relief space 122 is configured to enhance tissue contact with the cut electrode 106 during the cut cycle. Cupping occurs in devices with no relief space, so the resilient member 110 would push the tissue against the cut electrode 106 in mostly one direction, in such a way that the tissue gets pinched and arches (i.e., cups) around the edge 134 of the cut electrode 106, losing contact with the electrode edge 134.

**FIG. 7** illustrates a cross-sectional view of the interface between the end effector 105 of the medical device 100 and tissue 200 of a patient, in accordance with embodiments of the disclosed inventions. Selective components of the end effector 105 are partially shown in **FIG. 7** for clarity and better view of the tissue 200. As shown in **FIG. 7****,** the end effector 105 is in the closed configuration or substantially closed (e.g., jaws 102 and 104 near each other); the end effector 105 grasping, holding and/or restraining tissue 200 between jaws 102 and 104. The end effector 105 is configured to apply pressure and compress the tissue 200 disposed between the jaws 102 and 104, thereby creating compressed tissue portions 202 disposed between the sealing surfaces 130 and 132 and further creating a tented tissue portion 206 disposed between the support surface 108 and the cut electrode 106 (e.g., edge 134). Notably, as the tissue 200 is compressed by the end effector 105, tissue portions 204 (i.e., pillow) are pushed into the tissue reservoir 124 in both sides of the cut electrode 106, as shown in **FIG. 7****.**

The system 500 is configured to apply RF energy to the compressed tissue portion 202 in the seal cycle via the device 100. The power is selected and configured to limit current leakage beyond tissue that is not compressed between the seal surfaces 130 and 132, in a manner described in Applicant's owned US patents and applications, previously incorporated by reference herein.

In some embodiments, the system 500 is configured to apply a seal cycle having a maximum power of 55 Watts or less, a maximum voltage of 110 Volts rms or less, and a maximum current of 2.5 Amperes rms or less. In some embodiments, the seal cycle has a maximum power of 50 Watts or less. In some embodiments, the system 500 is configured to apply a cut cycle having a maximum power of 100 Watts or less, a maximum voltage of 380 Volts rms or less, and a maximum current of 2.5 Amperes rms nominal or less. In some embodiments, the cut cycle has a maximum voltage of 360 Volts rms nominal or less. In some embodiments, the cut cycle has a maximum power of 80 Watts nominal or less. The system 500 may be configured to allow the power, voltage, and current to float up to the maximum values in response to tissue impedance changes during the seal cycle.

In some embodiments, the system 500 is configured to seal tissue to a burst strength of at least three times systolic pressure. In some embodiments, the system 500 is configured to seal a vessel having a diameter of at least 7 millimeters to a burst strength of at least three times systolic pressure. In some embodiments, the system 500 is configured to cause thermal spread of 2 millimeters or less during the seal cycle. In some embodiments, the system 500 is configured to cause thermal spread of 1 millimeter or less during the seal cycle.

In some embodiments, the tissue in the compressed tissue portion 202 develops a higher impedance after sealing than it had before sealing, which affects the impedance between the cut electrode 106 and either the first seal surface 130 or the second seal surface 132. For example, prior to application of the seal cycle, the compressed tissue portion 202 typically has impedance of 100 Ohms or less, or between about 50 Ohms and about 100 Ohms. In some embodiments, it is desirable and efficient to provide a low-impedance path between the cut electrode 106 and the seal surface 130 or 132.

In some embodiments, the system 500 is configured to apply a cutting power to the tissue 200 retained between the jaws 102 and104 during the cut cycle, and not during the seal cycle. In some embodiments, the system 500 is configured to apply a seal cycle followed by a cut cycle. In some embodiments, a thermal relaxation time is provided between the seal cycle and the cut cycle. In these embodiments, the seal and cut cycles are sequential, where the cut cycle occurs after the seal cycle is performed. In alternative embodiments, the seal and cut cycles are performed simultaneously.

It should be apparent from **FIG. 7** that the end effector 105 is further configured to stretch (e.g., tent) the tissue 206 held between the pair of jaws against the cut electrode 106. The tenting may be achieved through the combination of the jaws 102 and 104 compressing tissue 200, as well as a slight contraction of tissue during a seal cycle and/or an effect of the support surface 108 and/or resilient member 110. Tenting of tissue 200 increases when the tissue 200 is slightly contracted by the seal cycle because the combined action of the cut electrode 106 with the support surface 108 and/or resilient member 110 acts like pulling the tissue 200 in one direction while the slight contraction of the seal cycle acts like pulling the tissue 200 in the opposite direction. The support surface 108 and/or resilient member 110 are configured to bias tissue 200 against the cut electrode 106 and toward the reservoir 124. The combination of tenting the tissue 206 and biasing tissue portions 204 into the tissue reservoir 124 during the cut cycle will inherently pull or move tissue away from the edge 134 of the cut electrode 106 as the tissue 200 is cut during the cut cycle, thereby promoting a monopolar tissue effect. Those skilled in the art know that monopolar operations such as tissue ablation require that the device remains in motion relative to the tissue being ablated - if the device stops the relative motion, further cutting of tissue is stopped and the tissue begins to desiccate and char; no cut. Currently, e-cutting devices on the market do not maintain this motion of tissue.

It should be appreciated that during the cut cycle, the tissue 200 clamped between jaws 102 and 104 begins to be electrically cut and divides from the cut electrode 106, so the jaws 102 and 104 move closer together to a substantially closed configuration of the end effector 105. The closing motion of the jaws 102 and 104 during the electrical cutting of tissue 200 causes the cut electrode 106 of the first jaw 102 to move closer to the second jaw 104, as the tissue 200 is cut, divides and separates. Additionally, as the tissue 200 is electrically cut and divides, the resilient member 110 exerts a restoring force returning the resilient member 110 to a prior shape and/or position (e.g., before the resilient member 110 was compressed by the tissue 200 and the cut electrode 106). The restoring force of the resilient member 110 is configured to push the tissue 200 towards the cut electrode 106, particularly the edge 134 of the cut electrode 106. The resilient member 110 comprises a high creep-recovery response, such that the resilient member 110 restoring force acts rapidly and does not lag or delay. A low creep-recovery response would cause undesirable loss of contact of the tissue 200 with the edge 134 of the cut electrode 106.

During the cut cycle, the cut electrode 106 of the first jaw 102 is electrically coupled with either the first seal surface 130 or the second seal surface 132 to cut tissue held between the jaws 102 and 104, such as cutting tissue tented between the cut electrode 106 and the non-conductive support surface 108. Therefore, the cut electrode 106 and the first or second seal surfaces 130, 132 are configured to conduct bipolar energy between the cut electrode 106 and the seal surfaces 130 or 132 during the cut cycle.

In some embodiments, during the cut cycle, the cut electrode 106 functions as an active electrode and the first or second seal surfaces 130, 132 functions as a dispersive electrode. In some embodiments, the cut electrode 106 has a relatively small exposed conductive surface, while the seal surface 130, 132 has a relatively large exposed conductive surface, whereby the current density drops significantly between the cut electrode 106 and the seal surface 130, 132, such that tissue near the seal surface 130, 132 is not damaged, while tented tissue portion 206 adjacent the edge 143 of the cut electrode 106 is cut.

In some embodiments, the cutting power has a bipolar energy, but the cutting power and the pair of jaws 102 and 104 are configured to create a monopolar power tissue effect to the tissue restrained between the pair of jaws. Although the energy conducted is bipolar, current flow from the cut electrode 106 towards either one of the seal surfaces 130 or 132 may have a monopolar effect during the cut cycle. Since, the current density is larger at the edge 134 of the cut electrode 106 than in either of the seal surfaces 130 or 132. Either of the seal surfaces 130 or 132 have a larger surface area compared to the smaller surface area of the edge 134, thus the seal surfaces 130 or 132 have a smaller current density than the edge 134 of the cut electrode 106.

The pillow tissue portions 204 comprise a relatively large area of low-impedance. The current density in the tented tissue portion 206 near the cut electrode edge 134 is higher than is the current density in the low-impedance tissue 204, effectively dispersing nearly all current and promoting current flow toward the seal surface 130 or the seal surface 132, rather than through pillow tissue portions 204, due to the seal surfaces 130 or 132 functioning as a dispersive electrode in a cut cycle.

The resulting current differential in the high-impedance tented tissue portion 206 near the edge 134 of the cut electrode 106 and the low-impedance pillow tissue portions 204 promotes cutting of tissue near the edge 134 of the cut electrode 106, rather than merely "cooking" or charring of tissue. In some embodiments, the system 500 may be configured to: (a) increase the impedance of compressed tissue portions 202 disposed between the jaws 102 and 104 during a seal cycle, (b) maintain a low impedance pillow tissue portions 204 during the seal cycle, and (c) subsequently apply a cutting power, with the cut electrode 106 being the active electrode, to cut tissue (e.g., tented tissue portion 206). In some embodiments, the system 500 is configured to apply a seal cycle, whereby tissue impedance is raised to an impedance of no more than 400 Ohms.

In some embodiments, the system 500 is configured to cut through 15 centimeters of mesentery tissue in 60 seconds or less, without leaving any tissue tags.

**FIGS. 8A-8B** illustrate cross-sectional views of the edge 134 of the cut electrode 106 including a graphical representation of current density during the cut cycle, according to embodiments of the disclosed inventions. **FIG. 8A** depicts a zoomed-in cross-sectional view of the edge 134 of the cut electrode 106 of **FIGS. 3****,** **4**, **5A-C** and **7** having a rounded, curved, and/or convex profile. As shown in **FIG. 8A****,** the cut electrode 106 is active during the cut cycle, such that current is distributed, substantially uniform, along the rounded conductive edge 134. As previously disclosed, the cut electrode 106 comprises insulation 136, making the sides of the cut electrode 106 non-conductive **(****FIGS. 8A-8B). FIG. 8B** illustrates an alternative edge 134" of the cut electrode 106, where the edge 134" comprises rounded corners 134a and a straight portion 134b therebetween. The alternative edge 134" is prone to create current concentrations at the corners 134a **(****FIG. 8B****);** thus, edge 134" includes a less uniform current distribution that the edge 134 of **FIG. 8A****.** In prior art devices, the edge of the cut electrode is sharp (not shown), which creates current concentrations at the sharp edge, reducing the effectiveness of the cutting power.

In some embodiments, the cut electrode 106 is shaped and configured to maximize a current concentration at the edge 134 of the cut electrode 106 during the cut cycle, while the second seal surface 132 is shaped and configured to minimize a current concentration at the seal surface 132 during the cut cycle. In some embodiments, the cut electrode 106 and the second seal surface 132 may be configured to create a monopolar tissue effect during a cut cycle, as previously disclosed. In alternative embodiments of the cut cycle, one of the seal surfaces 130 or 132 is exposed/conductive, while the other one is insulated, such that the exposed/conductive seal surface acts as a dispersive or return electrode of the cut electrode 106 during the cut cycle.

During the cut cycle, the second seal surface 132 or core member 118 may function as a dispersive electrode. In some embodiments, the first seal surface 130 or core member 114 may function as a dispersive electrode. In some embodiments, the first seal surface 130 and the second seal surface 132, or core members 114, 118, may both function as a dispersive electrode.

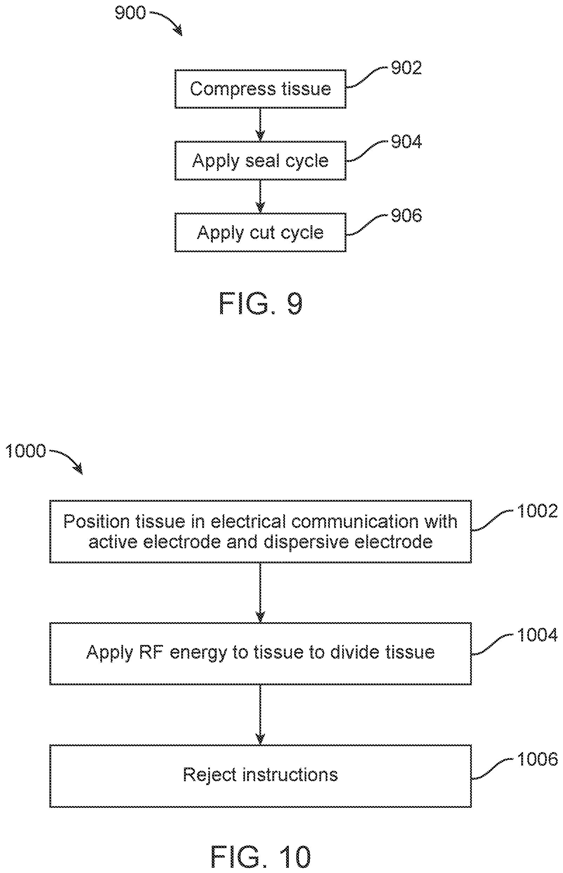

**FIG. 9** illustrates a method 900 for sealing and cutting tissue, according to the embodiments of the inventions. The medical system 500 is configured to be used in the manner, actions, and/or steps described by method 900.

The method 900 may include compressing 902 tissue grasped, held, disposed, and/or restrained between a pair of jaws of a medical device (e.g., surgical instrument). The method 900 may include applying a seal cycle 904 to the tissue held between the pair of jaws. The seal cycle having a maximum power of 55 Watts, to cause the tissue held between the pair of jaw to develop an impedance of 400 Ohms or less. After the seal cycle 904 is performed, the method 900 may include, applying a cut cycle 906 to the tissue held between the pair of jaws. The cut cycle having a maximum power of 100 Watts, to divide the tissue held between the pair of jaws, wherein the applying the cut cycle includes placing an active electrode and a dispersive electrode in contact with the tissue held between the pair of jaws. The seal cycle 906 may be configured to apply a sealing power selected to avoid causing thermal spread to tissue adjacent a cut electrode.

**FIG. 10** illustrates a method 1000 of dividing or cutting tissue, according to the embodiments of the inventions. The method 1000 may be performed by the system 500 described herein and/or may be a method of applying a cut cycle. The method 1000 may be performed in conjunction with method 900.

The method 1000 may include positioning 1002 tissue in electrical communication with an active electrode and a dispersive electrode. The method 1000 may include applying 1004 RF energy to the tissue to divide or severed the tissue. The RF energy having a maximum power of 100 Watts or less, a maximum current of 2.5 Amperes RMS or less, and a maximum voltage of 400 Volts rms or less.

In some embodiments, the applying RF energy to divide the tissue 1004 comprises dividing the tissue in 0.05 second or less. In some embodiments, the dividing the tissue 1004 comprises dividing the tissue in 0.125 seconds or less. In some embodiments, the dividing the tissue 1004 comprises dividing the tissue in 0.25 seconds or less. In some embodiments, the dividing the tissue 1004 comprises dividing the tissue in 1.5 seconds or less. In some embodiments, the dividing the tissue 1004 comprises dividing the tissue in 0.5 seconds or less. In some embodiments, the dividing the tissue 1004 comprises dividing the tissue in about 0.05 seconds, or in between about 0.02 seconds and about 0.1 seconds.

In some embodiments, the RF energy 1004 has a maximum power of 90 Watts. In some embodiments, the RF energy 1004 has a maximum power of 80 Watts nominal. In some embodiments, the RF energy 1004 has a maximum voltage of 380 Volts rms. In some embodiments, the RF energy 1004 has a maximum voltage of 360 Volts rms nominal.

In some embodiments, the power, voltage, and current are allowed to float up to the maximum power, maximum voltage, and maximum current in response to a change in an impedance of the tissue during the application of the cut cycle.

In some embodiments, the positioning the tissue 1002 comprises positioning tissue having a maximum impedance of 500 Ohms or less. In some embodiments, the positioning the tissue 1002 comprises positioning tissue having a maximum impedance of 400 Ohms or less. In some embodiments, all tissue providing electrical continuity between the active electrode such as the cut electrode 106 and the dispersive electrode such as seal surface 130, 132 or core member 114, 118 has an impedance of 1000 Ohms or less, or 500 Ohms or less, or 400 Ohms or less.

In some embodiments, the dividing the tissue 1004 comprises raising the impedance of at least a portion of the tissue in electrical continuity between the active electrode and the dispersive electrode to 5,000 Ohms or more. In some embodiments, the dividing the tissue 1004 comprises raising the impedance of the tissue to 10,000 Ohms or more.

It should be appreciated that the methods 900 and/or 1000 may include other actions and/or steps disclosed throughout this application and/or in the patents and applications incorporated by reference herein.

In some embodiments, the system 500 and/or method 900 may include applying a first cut cycle for a period of time of between about 80 milliseconds and about 120 milliseconds, or about 100 milliseconds, followed by a rest period of between about 80 milliseconds and about 120 milliseconds, followed by a second cut cycle for a period of time of between about 80 milliseconds and about 120 milliseconds, or about 100 milliseconds. The power algorithm described or incorporated by references herein typically completes the tissue cut in the first cut cycle, but the second cut cycle may be applied without harm, to confirm the first cut cycle was successful. The application of two cut cycles in this manner may insure a 100% cut success rate.

In some embodiments, the system 500 and method 900 may include applying a first seal cycle followed by two cut cycles and a second seal cycle, with a rest period between each cycle. In some embodiments, the second seal cycle may be followed by two cut cycles, with a rest period between each cycle. The rest periods may serve to limit thermal spread, thus improving the cut performance.

In some embodiments, the system 500 and/or method 900 and 1000 include a single generator or providing a single generator configured to output a seal cycle followed by a cut cycle. The device 100 may include three electrodes (e.g., seal surfaces 130, 132 and the cut electrode 106) or any other suitable number of electrodes configured to apply seal and cut cycles.

In some embodiments, the system 500 and/or method 900 and 1000 may include controlling a current density at or adjacent the cut electrode 106 during the cut cycle. The current density may be between about 100 Amperes per square inch and about 400 Amperes per square inch. The current density may be between 200 Amperes per square inch and about 400 Amperes per square inch. The current density may be greater than 100 Amperes per square inch during the application of the maximum power during the cut cycle. The current density may be greater than 200 Amperes per square inch during the application of the maximum power during the cut cycle.

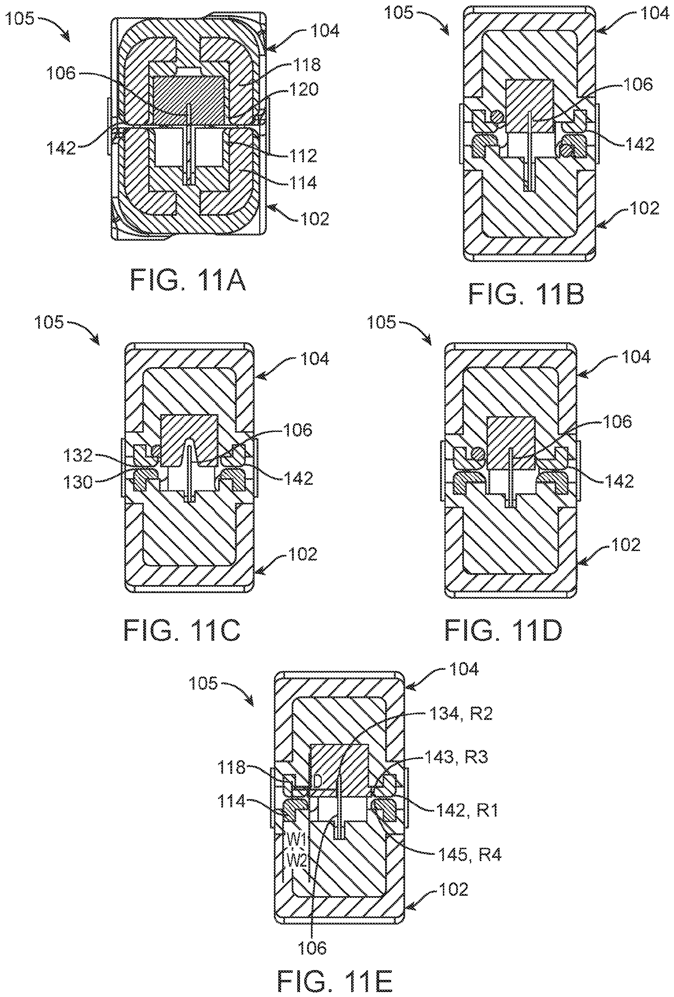

**FIGS. 11A-11E** illustrate alternative embodiments of the end effector 105 of the medical device, according to the disclosed inventions. As shown in **FIG. 6B** and **FIGS. 11A****-11E** the seal surface 132 of the second jaw 104 comprises the first rounded edge portion 142 having a radius R1 **(****FIG. 11E****).** The first rounded edge portion 142 may be shaped to limit current concentrations near the seal surface 132, such as, during the seal cycle. The radius R1 of the rounded edge portion 142 is configured to avoid current concentrations, thereby limiting tissue effects near the edge of the seal surface 132 during the seal cycle and/or during a cut cycle. Reducing current concentrations near the seal surface 132 may reduce thermal spread during the seal cycle. In some embodiments, the rounded edge portion 142 has a radius R1 of 0.254 millimeters or more. The first rounded edge portion 142 and radius R1 may be on an exterior wall (e.g., further away from the cut electrode 106) of the jaw 104.

Those skilled in the art will recognize that alternative end effector 105 and jaw configurations, such as **FIGS. 11A-11E****,** may be provided to avoid current concentrations on an interior portion (e.g., near the cut electrode 106) of one or both jaws 102, 104 during the cut cycle. Optionally, the end effector 105 may comprise cut electrodes 106 of various heights (e.g., **FIGS. 11B-11C****)** and/or various configurations of the core members 114, 118 and/or width of the seal surfaces 130, 132 **(****FIGS. 11A-11E****).**

For example, insulators 112, 120 may be provided on the inner/interior corners of the dispersive electrode(s) (e.g. core members 114, 118) or seal surface(s) 130, 132 of the jaw(s) 102, 104, so as to avoid current concentrations during the cut cycle, as shown in **FIG. 11A****,** and better appreciated in **FIGS. 5A-6C****.**

In another example, and as shown in **FIG. 11E****,** the seal surfaces 130, 132 or dispersive electrodes may include a second rounded edge portions 143, 145 having a radius R3, R4 selected to limit current concentrations near the surfaces 130, 132 during the cut cycle. In devices where the seal electrode edges are sharp, current concentration on the sharp edges can cause undesirable arcing, cutting and tissue sticking. In some embodiments, the second rounded edge portions 143, 145 of the dispersive electrodes are selected to have a radius R3, R4 that is 30% greater than a profile radius R2 of the edge 134 of the cut electrode 106, or more. In some embodiments, the rounded edge portions 143, 145 of the dispersive electrodes or core members 114, 118 are selected to have a radius R3, R4 that is 40% greater than a profile radius R2 of the edge 134 of the cut electrode 106, or more. In some embodiments, the rounded edge portions 143, 145 are selected to have a radius R3, R4 that is 50% greater than a profile radius R2 of the edge 134 of the cut electrode 106, or more. In some embodiments, the cut electrode 106 has a radius R2 of 0.006 inches or more.

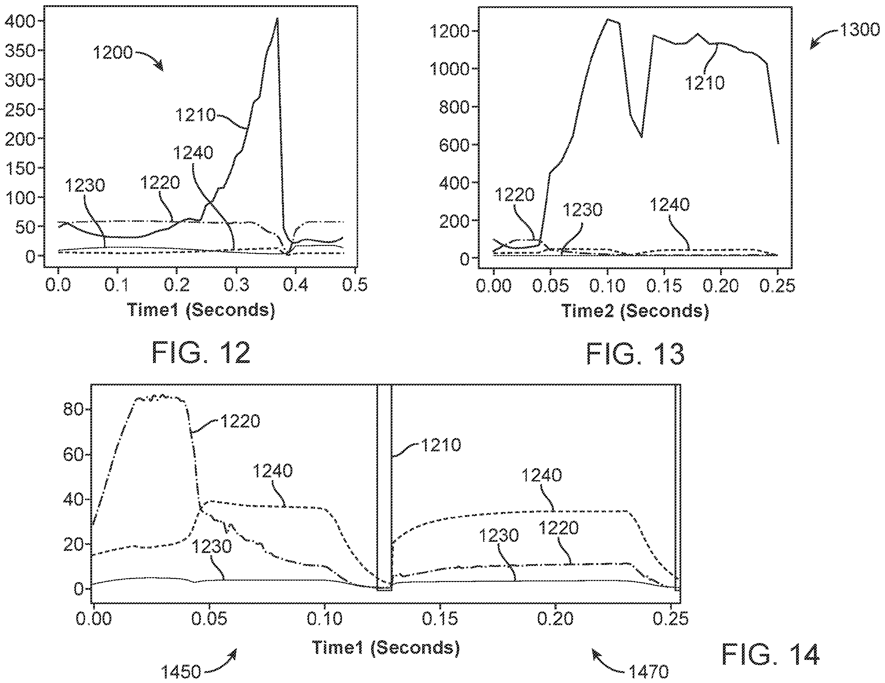

**FIG. 12** illustrates an exemplary seal cycle 1200 of the medical system 500, according to the disclosed inventions. When the system 500 is actuated and/or activated, such as by a user activating the actuator 300, which activates the power source 350, to perform the method 900, or 1000, the system 500 applies a seal cycle, shown at time zero. The time scale of **FIGS. 12-14** is seconds. In **FIGS. 12** and **14****,** the scale for power1 is times 1, the scale for voltage1 is times 0.1, the scale for current1 is times 10, and the scale for impedance1 is 1.

The system 500 is configured to detect the impedance 1210 of tissue compressed between the jaws 102 and 104 (e.g., compressed tissue 202 in **FIG. 7****).** While the tissue impedance 1210 is relatively low, the maximum power 1220, such as 50 Watts nominal **(****FIG. 12****),** may be reached and controlled. To maintain the maximum power 1220, the voltage 1240 and current 1230 may fluctuate in response to changes in the impedance 1210 of the compressed tissue 202 between the jaws 102 and 104.

When the system 500 detects that the impedance 1210 of the compressed tissue 202 has reached a threshold impedance level (e.g., a threshold between 400 to 500 Ohms), the system 500 is configured to discontinue applying the seal cycle 1200. In some embodiments, the time of the seal cycle 1200 may be 0.5 seconds or less. Optionally, the time of the seal cycle 1200 may be 1 second or less. Alternatively, the time of the seal cycle 1200 may be 2 seconds or less for tissue having a larger cross section.

After the seal cycle 1200 is applied to the tissue 200, the system 500 may allow a rest time before applying a cut cycle. The rest time may be between 50 milliseconds and 500 milliseconds.

**FIG. 13** illustrates an exemplary cut cycle 1300 of the system 500, according to the disclosed inventions. In **FIG. 13****,** the scale for power 2 is times 1, the scale for voltage2 is times 0.1, the scale for current2 is times 10, and the scale for impedance2 is times 0.1. After the seal cycle 1200 (or after the rest time), the system 500 may apply a cut cycle 1300 to the tissue restrained between the jaws 102 and 104; particularly, to the tented tissue portion 206 disposed between the support surface 108 and the cut electrode 106 (e.g., edge 134, as shown in **FIG. 7****).** The cut cycle 1300 starts at time zero, as shown in **FIG. 12****.** The cut cycle 1300 may be configured and applied as described with respect to method 900. The cut cycle 1300 may raise the tissue impedance 1210 significantly, such as to over 12,000 Ohms, as shown in **FIG. 13****.** Alternatively, the cut cycle 1300 may raise the tissue impedance 1210 such as to 5,000 Ohms or more, or 10,000 Ohms or more, or to 50,000 Ohms or more. The system 500 is configured to discontinue the cut cycle 1300 when the tissue impedance 1210 is raised at the limits previously described, and/or after approximately 0.100 seconds to 0.125 seconds after starting the cut cycle 1300. The system 500 may stop the cut cycle after a successful cut, as shown in **FIG. 13** by the quick drop in impedance 1210 at about 0.1 seconds. The system 500 may apply a second cut cycle, such as to confirm the first cut cycle was successful, shown in **FIG. 13** starting at about 0.125 seconds, shown by the quick rise in impedance 1210 after the drop.

In some embodiments, the system 500 is configured to maintain a substantial constant power 1220 during the seal cycle and/or cut cycle. In order to maintain the substantial constant power 1220, the system 500 will increase voltage 1240 and decrease current 1230 or decrease voltage 1240 and increase current 1230, in response to changes in the impedance 1210 of the compressed tissue 202 between the jaws 102 and 104. For example, as shown in **FIG. 12****,** when impedance 1210 drops, voltage 1240 is increased and current 1230 is decreased, so the slight drop in power 1220 is adjusted back to remain substantially constant.

**FIG. 14** illustrates a detailed view of the exemplary cut cycle 1300 of **FIG. 13****,** according to the disclosed inventions. **FIG. 14** graph omits portions of the impedance 1210. As shown in **FIG. 14****,** during a first cut cycle 1450, the system 500 will quickly increase power 1220 to a maximum power (approximately 80 watts) while voltage 1240 is relatively low. As the impedance 1210 of the tissue changes, however, the power 1220 will drop and the voltage 1240 will increase. If the first cut cycle 1450 is successful, a second cut cycle 1470 will result in high impedance 1210, with the system 500 at maximum voltage 1240, maximum power 1220, and low current 1230. Note the difference between the power 1220 output shown in **FIG. 14** before the impedance 1210 drops (e.g., signaling a break between the first cut cycle and the second cut cycle) and after the impedance 1210 is raised once more.

In some embodiments, the power 1220, voltage 1240, and current 1230 are allowed to float up to the maximum power, maximum voltage, and maximum current in response to a change in impedance 1210 of the tissue 200 during the application of the cut cycle.

Although particular embodiments have been shown and described herein, it will be understood by those skilled in the art that they are not intended to limit the present inventions, and it will be obvious to those skilled in the art that various changes, permutations, and modifications may be made (e.g., the dimensions of various parts, combinations of parts) without departing from the scope of the disclosed inventions, which is to be defined only by the following claims .

## Claims

1. A surgical system (500) for sealing and severing tissue, the system comprising:
a generator (350) configured to output radiofrequency, RF, energy, including delivering a first RF power signal during a seal cycle, and delivering a second RF power signal during a cut cycle; and
a surgical device (100) electrically coupled to the generator output, the device comprising
an elongate shaft (113);
an end effector (105) coupled to a distal end portion of the elongate shaft (113), wherein the elongate shaft defines a longitudinal axis (152), and wherein the end effector comprises first and second jaws (102, 104) configured to approximate each other in a closed position for compressing tissue (200) extending therebetween, the first and second jaws (102, 104) having respective opposing conductive seal surface electrodes (130, 132) configured for sealing spaced apart first and second portions of the compressed tissue (202) when the first RF power signal is conducted through a circuit including the respective seal surface electrodes (130, 132) during a seal cycle; and
a cut electrode (106) disposed within an interior region of the first jaw (102), wherein the cut electrode (106) is aligned with the longitudinal axis and has an elongate conductive edge (134) with a convex cross-section profile taken perpendicular to the longitudinal axis (152), the cut electrode having a height profile greater than the seal surface electrodes (130, 132) of the first jaw (102), and wherein when the first and second jaws (102, 104) are in the closed position compressing the tissue, the elongate conductive edge (134) of the cut electrode (106) presses against a third portion of the tissue (206) located between the spaced apart first and second portions (202),
wherein the cut electrode (106) is configured to sever the third portion of the tissue (206) when the second RF power signal is conducted through a circuit including the cut electrode (106) and a respective one of the seal surface electrodes (130, 132) during a cut cycle.

2. The surgical system (500) of claim 1, further comprising a sensing circuit for detecting parameters indicative of an impedance of the compressed tissue (202), wherein during the seal cycle, power, voltage, and current are allowed to float up to a sealing power of no more than 55 Watts, a current of no more than 2.5 Amperes rms, and a voltage of 110 Volts rms in response to changes in the impedance of the compressed tissue (202).

3. The surgical system (500) of claim 1 or 2, further comprising a sensing circuit for detecting parameters indicative of an impedance of the tissue (200), wherein during the cut cycle, power, voltage, and current are allowed to float up to a cutting power of no more than 80 Watts, a current of no more than 2.5 Amperes rms, and a voltage of 380 Volts rms in response to changes in the impedance of the compressed tissue (202).

4. The surgical system (500) of any of claims 1-3, wherein the generator maintains power below a maximum seal power and a maximum cut power by varying current or voltage in response to changes in impedance of the tissue (200) during respective seal and cut cycles wherein the maximum cut power is greater than the maximum seal power.

5. The surgical system (500) of any of claims 1-4, wherein, the elongate conductive edge (134) of the cut electrode (106) is configured to provide a substantially uniform current concentration during the cut cycle.

6. The surgical system (500) of any of claims 1-5, wherein the cut cycle is initiated after completion of the seal cycle.

7. The surgical system (500) of any of claims 1-6, wherein when the first and second jaws (102, 104) are in the closed position compressing the tissue (200), the elongate conductive edge (134) of the cut electrode (106) stretches the third portion of the tissue.

8. The surgical system (500) of any of claims 1-7, further comprising a resilient member (110) disposed in a cavity (121) defined by the second jaw opposing (104) the elongate conductive edge (134) of the cut electrode (106), wherein when the first and second jaws (102, 104) are in the closed position compressing the tissue (200), the elongate conductive edge and the resilient member are configured to compress the third portion of the tissue therebetween.

9. The surgical system (500) of claim 8, the resilient member (110) having a bias against the elongate conductive edge (134) of the cut electrode (106) so as to impose a restoring force that presses the third portion of the tissue against the elongate conductive edge of the cut electrode during the cut cycle.

10. The surgical system (500) of claim 8 or 9, wherein the cavity (121) comprises a relief space (122), wherein the relief space (122) is configured to receive at least a portion of the resilient member (110) when the first and second jaws (102, 104) are in the closed position and compressing the tissue.

11. The surgical system (500) of any of claims 8-10, wherein the resilient member (110) comprises a non-conductive support surface (108) that contacts the third portion of the tissue.

12. The surgical system (500) of any of claims 1-11, wherein the at least one of the seal surface electrodes (130, 132) comprises a seal surface profile having a tissue compression portion and a first rounded edge portion, wherein the first rounded edge portion is configured to minimize current concentrations during the cut cycle.

13. The surgical system (500) of any of claims 1-12, wherein the respective conductive seal surface electrodes (130, 132) of the first and second jaws (102, 104) are configured to impart a bi-polar effect on the respective first and second portions of the tissue during the seal cycle.

14. The surgical system (500) of any of claims 1-13, wherein the respective one of the seal surface electrode (130, 132) that forms the cut cycle circuit with the cut electrode acts as a dispersive electrode such that the cut electrode imparts a monopolar effect on the third portion of the tissue during a cut cycle.

15. The surgical system (500) of any of claims 1-14, wherein the interior region of the first jaw (102) defines at least one tissue reservoir (124) disposed adjacent the cut electrode (106), wherein the tissue reservoir is configured to receive prolapsed tissue when the first and second jaws (102, 104) are in the closed position and compressing the tissue.

## Patentansprüche

1. Chirurgisches System (500) zum Versiegeln und Durchtrennen von Gewebe, wobei das System Folgendes umfasst:
einen Generator (350), der dazu konfiguriert ist, Hochfrequenzenergie, HF-Energie, auszugeben, was Liefern eines ersten HF-Leistungssignals während eines Versiegelungszyklus und Liefern eines zweiten HF-Leistungssignals während eines Schneidezyklus enthält; und
eine chirurgische Vorrichtung (100), die elektrisch mit dem Generatorausgang gekoppelt ist, wobei die Vorrichtung einen länglichen Schaft (113) umfasst;
einen Endeffektor (105), der mit einem distalen Endabschnitt des länglichen Schafts (113) gekoppelt ist, wobei der längliche Schaft eine Längsachse (152) definiert, und wobei der Endeffektor eine erste und eine zweite Backe (102, 104) umfasst, die dazu konfiguriert sind, sich in einer geschlossenen Position einander anzunähern, um sich dazwischen erstreckendes Gewebe (200) zu komprimieren, wobei die erste und die zweite Backe (102, 104) jeweilige gegenüberliegende leitende Versiegelungsoberflächenelektroden (130, 132) aufweisen, die dazu konfiguriert sind, einen ersten und einen zweiten Abschnitt des komprimierten Gewebes (202), die voneinander beabstandet sind, zu versiegeln, wenn das erste HF-Leistungssignal während eines Versiegelungszyklus durch einen Schaltkreis geleitet wird, der die jeweiligen Versiegelungsoberflächenelektroden (130, 132) enthält; und
eine Schneidelektrode (106), die in einem Innenbereich der ersten Backe (102) angeordnet ist, wobei die Schneidelektrode (106) auf die Längsachse ausgerichtet ist und eine längliche leitende Kante (134) mit einem konvexen Querschnittsprofil senkrecht zur Längsachse (152) aufweist, wobei die Schneidelektrode ein Höhenprofil aufweist, das größer als die Versiegelungsoberflächenelektroden (130, 132) der ersten Backe (102) ist, und wobei, wenn sich die erste und die zweite Backe (102, 104) in der geschlossenen Position befinden und das Gewebe komprimieren, die längliche leitende Kante (134) der Schneidelektrode (106) gegen einen dritten Abschnitt des Gewebes (206) drückt, der sich zwischen dem ersten und dem zweiten Abschnitt (202), die beabstandet sind, befindet,
wobei die Schneidelektrode (106) dazu konfiguriert ist, den dritten Abschnitt des Gewebes (206) zu durchtrennen, wenn das zweite HF-Leistungssignal während eines Schneidezyklus durch einen Schaltkreis geleitet wird, der die Schneidelektrode (106) und eine jeweilige der Versiegelungsoberflächenelektroden (130, 132) enthält.

2. Chirurgisches System (500) nach Anspruch 1, ferner einen Sensorschaltkreis zum Erkennen von Parametern umfassend, die eine Impedanz des komprimierten Gewebes (202) angeben, wobei während des Versiegelungszyklus erlaubt ist, dass Leistung, Spannung und Strom als Reaktion auf Änderungen der Impedanz des komprimierten Gewebes (202) bis auf eine Versiegelungsleistung von nicht mehr als 55 Watt, einen Strom von nicht mehr als 2,5 Ampere effektiv und eine Spannung von 110 Volt effektiv schwanken.

3. Chirurgisches System (500) nach Anspruch 1 oder 2, ferner einen Sensorschaltkreis zum Erkennen von Parametern umfassend, die eine Impedanz des Gewebes (200) angeben, wobei während des Schneidezyklus erlaubt ist, dass Leistung, Spannung und Strom als Reaktion auf Änderungen der Impedanz des komprimierten Gewebes (202) bis auf eine Schneidleistung von nicht mehr als 80 Watt, einen Strom von nicht mehr als 2,5 Ampere effektiv und eine Spannung von 380 Volt effektiv schwanken.

4. Chirurgisches System (500) nach einem der Ansprüche 1-3, wobei der Generator eine Leistung unterhalb einer maximalen Versiegelungsleistung und einer maximalen Schneidleistung aufrechterhält, indem er einen Strom oder eine Spannung als Reaktion auf Änderungen der Impedanz des Gewebes (200) während jeweiliger Versiegelungs- und Schneidezyklen variiert, wobei die maximale Schneidleistung größer als die maximale Versiegelungsleistung ist.

5. Chirurgisches System (500) nach einem der Ansprüche 1-4, wobei die längliche leitende Kante (134) der Schneidelektrode (106) dazu konfiguriert ist, während des Schneidezyklus eine im Wesentlichen gleichmäßige Stromkonzentration bereitzustellen.

6. Chirurgisches System (500) nach einem der Ansprüche 1-5, wobei der Schneidezyklus nach Abschluss des Versiegelungszyklus eingeleitet wird.

7. Chirurgisches System (500) nach einem der Ansprüche 1-6, wobei die längliche leitende Kante (134) der Schneidelektrode (106) den dritten Abschnitt des Gewebes dehnt, wenn sich die erste und die zweite Backe (102, 104) in der geschlossenen Position befinden und das Gewebe (200) komprimieren.

8. Chirurgisches System (500) nach einem der Ansprüche 1-7, ferner ein elastisches Element (110) umfassend, das in einem Hohlraum (121) angeordnet ist, der durch die zweite Backe definiert ist und der länglichen leitenden Kante (134) der Schneidelektrode (106) gegenüberliegt (104), wobei die längliche leitende Kante und das elastische Element dazu konfiguriert sind, den dritten Abschnitt des Gewebes dazwischen zu komprimieren, wenn sich die erste und die zweite Backe (102, 104) in der geschlossenen Position befinden und das Gewebe (200) komprimieren.

9. Chirurgisches System (500) nach Anspruch 8, wobei das elastische Element (110) eine Vorspannung gegen die längliche leitende Kante (134) der Schneidelektrode (106) aufweist, um eine Rückstellkraft auszuüben, die den dritten Abschnitt des Gewebes während des Schneidzyklus gegen die längliche leitende Kante der Schneidelektrode drückt.

10. Chirurgisches System (500) nach Anspruch 8 oder 9, wobei der Hohlraum (121) einen Aussparungsraum (122) umfasst, wobei der Aussparungsraum (122) dazu konfiguriert ist, mindestens einen Abschnitt des elastischen Elements (110) aufzunehmen, wenn sich die erste und die zweite Backe (102, 104) in der geschlossenen Position befinden und das Gewebe komprimieren.

11. Chirurgisches System (500) nach einem der Ansprüche 8-10, wobei das elastische Element (110) eine nichtleitende Stützoberfläche (108) umfasst, die den dritten Abschnitt des Gewebes berührt.

12. Chirurgisches System (500) nach einem der Ansprüche 1-11, wobei die mindestens eine der Versiegelungsoberflächenelektroden (130, 132) ein Versiegelungsoberflächenprofil mit einem Gewebekompressionsabschnitt und einem ersten abgerundeten Kantenabschnitt umfasst, wobei der erste abgerundete Kantenabschnitt dazu konfiguriert ist, Stromkonzentrationen während des Schneidzyklus zu minimieren.

13. Chirurgisches System (500) nach einem der Ansprüche 1-12, wobei die jeweiligen leitenden Versiegelungsoberflächenelektroden (130, 132) der ersten und der zweiten Backe (102, 104) dazu konfiguriert sind, während des Versiegelungszyklus eine bipolare Wirkung auf den jeweiligen ersten und zweiten Abschnitt des Gewebes auszuüben.

14. Chirurgisches System (500) nach einem der Ansprüche 1-13, wobei die jeweilige der Versiegelungsoberflächenelektrode (130, 132), die den Schneidezyklusschaltkreis mit der Schneidelektrode ausbildet, als eine Neutralelektrode fungiert, sodass die Schneidelektrode während eines Schneidezyklus eine monopolare Wirkung auf den dritten Abschnitt des Gewebes ausübt.

15. Chirurgisches System (500) nach einem der Ansprüche 1-14, wobei der Innenbereich der ersten Backe (102) mindestens ein Gewebereservoir (124) definiert, das angrenzend an die Schneidelektrode (106) angeordnet ist, wobei das Gewebereservoir dazu konfiguriert ist, vorgefallenes Gewebe aufzunehmen, wenn sich die erste und die zweite Backe (102, 104) in der geschlossenen Position befinden und das Gewebe komprimieren.

## Revendications

1. Système chirurgical (500) pour sceller et sectionner des tissus, le système comprenant :
un générateur (350) conçu pour sortir de l'énergie radiofréquence, RF, comprenant la délivrance d'un premier signal de puissance RF pendant un cycle de scellement, et la délivrance d'un second signal de puissance RF pendant un cycle de coupe ; et
un dispositif chirurgical (100) couplé électriquement à la sortie du générateur, le dispositif comprenant
une tige allongée (113) ;
un effecteur terminal (105) couplé à une partie d'extrémité distale de la tige allongée (113), dans lequel la tige allongée définit un axe longitudinal (152), et dans lequel l'effecteur terminal comprend des première et seconde mâchoires (102, 104) configurées pour se rapprocher l'une de l'autre dans une position fermée pour comprimer le tissu (200) s'étendant entre elles, les première et seconde mâchoires (102, 104) comportant des électrodes de surface de scellage conductrices opposées respectives (130, 132) conçues pour sceller des première et deuxième parties espacées du tissu comprimé (202) lorsque le premier signal de puissance RF est conduit à travers un circuit comprenant les électrodes de surface de scellage respectives (130, 132) pendant un cycle de scellage ; et
une électrode de coupe (106) disposée dans une région intérieure de la première mâchoire (102), dans lequel l'électrode de coupe (106) est alignée avec l'axe longitudinal et comporte un bord conducteur allongé (134) comportant un profil de section transversale convexe pris perpendiculairement à l'axe longitudinal (152), l'électrode de coupe comportant un profil de hauteur supérieur à celui des électrodes de surface de scellage (130, 132) de la première mâchoire (102), et dans lequel lorsque les première et seconde mâchoires (102, 104) sont en position fermée comprimant le tissu, le bord conducteur allongé (134) de l'électrode de coupe (106) appuie contre une troisième partie du tissu (206) située entre les première et deuxième parties espacées (202),
dans lequel l'électrode de coupe (106) est conçue pour sectionner la troisième partie du tissu (206) lorsque le second signal de puissance RF est conduit à travers un circuit comprenant l'électrode de coupe (106) et l'une respective des électrodes de surface de scellage (130, 132) pendant un cycle de coupe.

2. Système chirurgical (500) selon la revendication 1, comprenant en outre un circuit de détection pour détecter des paramètres indicatifs d'une impédance du tissu comprimé (202), dans lequel pendant le cycle de scellage, la puissance, la tension et le courant sont autorisés à flotter jusqu'à une puissance de scellage ne dépassant pas 55 watts, un courant ne dépassant pas 2,5 ampères rms et une tension de 110 volts rms en réponse aux changements de l'impédance du tissu comprimé (202).

3. Système chirurgical (500) selon la revendication 1 ou 2, comprenant en outre un circuit de détection pour détecter des paramètres indicatifs d'une impédance du tissu (200), dans lequel pendant le cycle de coupe, la puissance, la tension et le courant sont autorisés à flotter jusqu'à une puissance de coupe ne dépassant pas 80 watts, un courant ne dépassant pas 2,5 ampères rms et une tension de 380 volts rms en réponse aux changements de l'impédance du tissu comprimé (202).

4. Système chirurgical (500) selon l'une quelconque des revendications 1 à 3, dans lequel le générateur maintient la puissance en dessous d'une puissance de scellage maximale et d'une puissance de coupe maximale en faisant varier le courant ou la tension en réponse aux changements d'impédance du tissu (200) pendant les cycles de scellage et de coupe respectifs, dans lequel la puissance de coupe maximale est supérieure à la puissance de scellage maximale.

5. Système chirurgical (500) selon l'une quelconque des revendications 1 à 4, dans lequel le bord conducteur allongé (134) de l'électrode de coupe (106) est conçu pour fournir une concentration de courant sensiblement uniforme pendant le cycle de coupe.

6. Système chirurgical (500) selon l'une quelconque des revendications 1 à 5, dans lequel le cycle de coupe est initié après l'achèvement du cycle de scellage.

7. Système chirurgical (500) selon l'une quelconque des revendications 1 à 6, dans lequel lorsque les première et seconde mâchoires (102, 104) sont en position fermée comprimant le tissu (200), le bord conducteur allongé (134) de l'électrode de coupe (106) étire la troisième partie du tissu.

8. Système chirurgical (500) selon l'une quelconque des revendications 1 à 7, comprenant en outre un élément élastique (110) disposé dans une cavité (121) définie par la seconde mâchoire (104) opposée au bord conducteur allongé (134) de l'électrode de coupe (106), dans lequel, lorsque les première et seconde mâchoires (102, 104) sont dans la position fermée comprimant le tissu (200), le bord conducteur allongé et l'élément élastique sont configurés pour comprimer la troisième partie du tissu entre eux.

9. Système chirurgical (500) selon la revendication 8, l'élément élastique (110) comportant un rappel contre le bord conducteur allongé (134) de l'électrode de coupe (106) de manière à imposer une force de restauration qui presse la troisième partie du tissu contre le bord conducteur allongé de l'électrode coupée pendant le cycle de coupe.

10. Système chirurgical (500) selon la revendication 8 ou 9, dans lequel la cavité (121) comprend un espace de décharge (122), dans lequel l'espace de décharge (122) est configuré pour recevoir au moins une partie de l'élément élastique (110) lorsque les première et seconde mâchoires (102, 104) sont en position fermée et compriment le tissu.

11. Système chirurgical (500) selon l'une quelconque des revendications 8 à 10, dans lequel l'élément élastique (110) comprend une surface de support non conductrice (108) qui vient en contact avec la troisième partie du tissu.

12. Système chirurgical (500) selon l'une quelconque des revendications 1 à 11, dans lequel l'au moins une des électrodes de surface de scellage (130, 132) comprend un profil de surface de scellage comportant une partie de compression de tissu et une première partie de bord arrondi, dans lequel la première partie de bord arrondi est conçue pour minimiser les concentrations de courant pendant le cycle de coupe.

13. Système chirurgical (500) selon l'une quelconque des revendications 1 à 12, dans lequel les électrodes de surface de scellage conductrices respectives (130, 132) des première et seconde mâchoires (102, 104) sont conçues pour conférer un effet bipolaire sur les première et deuxième parties respectives du tissu pendant le cycle de scellage.

14. Système chirurgical (500) selon l'une quelconque des revendications 1 à 13, dans lequel celle respective de l'électrode de surface de scellage (130, 132) qui forme le circuit de cycle de coupe avec l'électrode de coupe agit comme une électrode dispersive de telle sorte que l'électrode de coupe confère un effet monopolaire sur la troisième partie du tissu pendant un cycle de coupe.

15. Système chirurgical (500) selon l'une quelconque des revendications 1 à 14, dans lequel la région intérieure de la première mâchoire (102) définit au moins un réservoir de tissu (124) disposé adjacent à l'électrode de coupe (106), dans lequel le réservoir de tissu est configuré pour recevoir du tissu prolabé lorsque les première et seconde mâchoires (102, 104) sont en position fermée et compriment le tissu.
